(19) Europäisches Patentamt
European Patent Office
Office européen des brevets

(11) EP 2 727 594 B1

(12) **EUROPEAN PATENT SPECIFICATION**

(45) Date of publication and mention of the grant of the patent:
**20.12.2017 Bulletin 2017/51**

(21) Application number: **12803608.4**

(22) Date of filing: **26.06.2012**

(51) Int Cl.:
***A61K 31/4709*** (2006.01) ***A61K 31/502*** (2006.01)
***A61P 17/04*** (2006.01) ***C07D 401/04*** (2006.01)
***A61P 43/00*** (2006.01)

(86) International application number:
**PCT/JP2012/066220**

(87) International publication number:
**WO 2013/002196 (03.01.2013 Gazette 2013/01)**

(54) **NAPHTALENE COMPOUNDS TO TREAT ITCH**

NAPHTALENE VERBINDUNGEN ZUR BEHANDLUNG VON JUCKREIZ

COMPOSÉS NAPHTALÉNIQUES POUR TRAITER LES DÉMANGEAISONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2011 JP 2011143283**

(43) Date of publication of application:
**07.05.2014 Bulletin 2014/19**

(73) Proprietors:
- **Mitsubishi Tanabe Pharma Corporation**
**Osaka 541-8505 (JP)**
- **Maruho Co., Ltd.**
**Osaka 531-0071 (JP)**

(72) Inventors:
- **OONO, Ryoko**
**Osaka-shi,**
**Osaka 541-8505 (JP)**
- **KIDO, Hiroko**
**Kyoto-shi**
**Kyoto 600-8815 (JP)**

(74) Representative: **Hoffmann Eitle**
**Patent- und Rechtsanwälte PartmbB**
**Arabellastraße 30**
**81925 München (DE)**

(56) References cited:
**WO-A1-2007/043426**
**JP-A- 9 059 255**
**JP-A- 10 226 647**
**JP-A- 2001 520 196**
**JP-A- 2005 047 909**
**JP-A- 2010 280 622**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 727 594 B1

## Description

TECHNICAL FIELD

The present invention relates to a novel pharmaceutical composition (antipruritic agent) which is useful for prophylaxis or treatment of itch not induced by skin inflammatory reactions.

BACKGROUND ART

Itch is a distressing symptom that may worsen quality of life (QOL) of both patients suffered from inflammatory skin diseases such as atopic dermatitis and patients suffered from diseases not associated with skin inflammation such as skin pruritus. Besides, itch-induced scratching behavior may leads to damages in barrier function of skin, secondary skin lesions and progression of said disease. Therefore, inhibition of itch is a very important problem in treatment of the above mentioned diseases. Whereas a drug such as a steroid, an immunosuppressant, an antihistaminic agent or the like is frequently prescribed for the treatment of the diseases as above, these drugs are not sufficiently effective in suppressing itch. Therefore, more effective drugs are desired to be developed.

On the other hand, it has been known that a naphthalene compound as an active ingredient in the present invention possesses PDE4 inhibitory activity and the compound is thereby useful as an anti-asthmatic agent, a dermatitis treating agent and the like (Patent Literature 1 and 2). However, such background arts neither disclose nor suggest whether or not the naphthalene compounds as above are effectively inhibit various sorts of itch such as that in atopic dermatitis, psoriasis, skin pruritus or chronic prurigo. There have been some reports that some PDE4 inhibitors are effective in suppressing itch associated with inflammatory diseases (Patent literature 3 and 4). However, the mechanism in suppressing itch remains unclear.

Under such situation, throughout the intensive studies, the present inventors found that some specific naphthalene compounds effectively inhibit itch induced by inflammatory reactions and also itch which is not induced by inflammatory reactions, and thereby completed the present invention.

BACKGROUND ART DOCUMENTS

Patent literatures

Patent literature 1: EP0748805A1
Patent literature 2: WO2007/043426
Patent literature 3: JP2005-47909
Patent literature 4: WO99/020280

SUMMARY OF THE INVENTION

PROBLEMS TO BE SOLVED BY THE INVENTION

The present invention provides a novel pharmaceutical composition (antipruritic agent) useful as an agent for prophylaxis or treatment of itch (atopic dermatitis, psoriasis, chronic pruritus, skin pruritus or the like) not induced by skin inflammatory reactions.

MEANS FOR SOLVING THE PROBLEMS

The present invention provides

(1) a pharmaceutical composition for use in the treatment or prophylaxis of itch not induced by skin inflammatory reactions comprising as an active ingredient a naphthalene compound of the following formula (I):

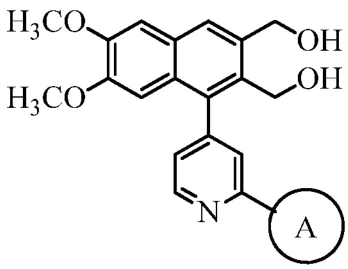

(I)

wherein the ring A is a heterocyclic group of the following formula:

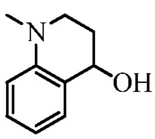 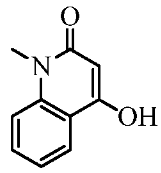 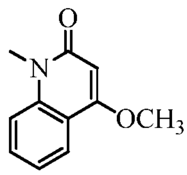 or 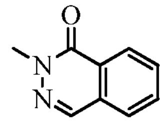 ,

a hydrate thereof or a pharmaceutically acceptable salt thereof;

(2) the pharmaceutical composition for use according to the embodiment (1) mentioned above in which the ring A is a heterocyclic group of the following formula:

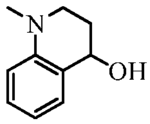

;

(3) the pharmaceutical composition for use according to the embodiment (1) mentioned above in which the ring A is a heterocyclic group of the following formula:

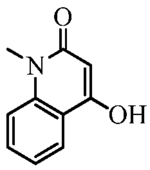

;

(4) the pharmaceutical composition for use according to the embodiment (1) mentioned above in which the ring A is a heterocyclic group of the following formula:

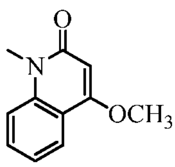

;

(5) the pharmaceutical composition for use according to the embodiment (1) mentioned above in which the ring A is a heterocyclic group of the following formula:

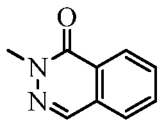;

(6) the pharmaceutical composition for use according to the embodiment (1) mentioned above in which the ring A is a heterocyclic group of the following formula:

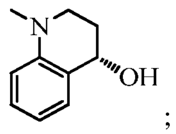

;

(7) the pharmaceutical composition for use according to the embodiment (1) mentioned above in which the ring A is a heterocyclic group of the following formula:

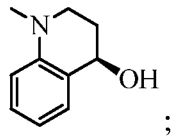

;

(8) a pharmaceutical composition for use in the treatment of itch not induced by skin inflammatory reactions comprising as an active ingredient a compound of the following formula (I-A):

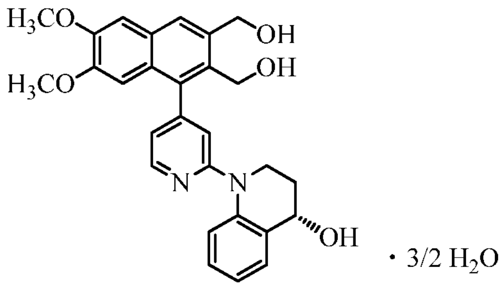

(I-A)

;

(9) the pharmaceutical composition for use according to either one of the above-described embodiments (1) to (8) which is an agent for treatment of itch not induced by skin inflammatory reactions;
(10) the pharmaceutical composition for use according to the above-described embodiment (9) which is an agent for prophylaxis or treatment of pruritus;
(11) the pharmaceutical composition for use according to the above-described embodiment (1) which is an agent for prophylaxis or treatment of chronic prurigo;
(12) the pharmaceutical composition for use according to the above-described embodiment (10) in which the pruritus is that associated with primary biliary cirrhosis, chronic renal failure/renal dialysis, abnormal blood pressure, thyroid gland malfunction, aging, cancer, anemia, a parasitic disease, a psycho-neurologic disease, a drug-induced disease and/or pregnancy, or pruritogen-induced pruritus;
(13) the pharmaceutical composition for use according to the above-described embodiment (9) which is an agent for inhibiting itch resistant to a steroid, an anti-inflammatory agent or an immunosuppressant;
(16) a naphthalene compound of the above formula (I) for use in the prophylaxis or treatment of itch not induced by skin inflammatory reactions which comprises topically administering a prophylactically or therapeutically effective amount of the naphthalene compound of the above formula (I), a hydrate thereof or a pharmaceutically acceptable salt thereof to a patient in need thereof;
(17) a naphthalene compound of the above formula (I), a hydrate thereof or a pharmaceutically acceptable salt thereof for use in in the prophylaxis or treatment of itch not induced by skin inflammatory reactions; and
(18) use of a naphthalene compound of the above formula (I), a hydrate thereof or a pharmaceutically acceptable salt thereof for the manufacture of a medicament which is used for prophylaxis or treatment of itch not induced by skin inflammatory reactions.

EFFECT OF THE INVENTION

The pharmaceutical composition (antipruritic agent) of the present invention shows an excellent inhibiting effect on itch induced by various causes. Concretely, the pharmaceutical composition of the present invention is useful for

prophylaxis or treatment of itch that is not associated with inflammatory skin reactions, such as pruritus associated with primary biliary cirrhosis, chronic renal failure/renal dialysis, abnormal blood pressure, thyroid gland malfunction, aging, cancer, anemia, a parasitic disease, a psycho-neurologic disease, a drug-induced disease and/or pregnancy, or pruritus induced by a pruritogen such as histamine, and further useful for prophylaxis or treatment of intractable itch associated with chronic prurigo etc. Besides, the pharmaceutical composition (antipruritic agent) of the present invention is useful for inhibiting itch which is resistant to a conventional medicament such as a steroid, an anti-inflammatory agent (e.g., an antihistamine) or an immunosuppressant (e.g., tacrolimus).

The compound (I), a hydrate thereof or a pharmaceutically acceptable salt thereof as an active ingredient of the present invention includes that does not produce toxicity or an adverse reaction such as local stimulus, skin photosensitization, central nervous system depressant action (e.g., inhibitory effect on spontaneous locomotor activity etc.) at least within the effective dose as an antipruritic agent. The pharmaceutical composition (antipruritic agent) of the present invention comprising such a compound, a hydrate thereof or a pharmaceutically acceptable salt thereof as an active ingredient is useful from an aspect of safety.

EMBODIMENTS TO CARRY OUT THE INVENTION

When the compound (I) as an active ingredient of the present invention has an asymmetric carbon atom(s) in its molecule, it may exist in the form of a stereoisomer thereof (diastereoisomers, optical isomers) owing to said asymmetric carbon atom(s) thereof, and the active ingredient of the present invention also includes one of the stereoisomers and a mixture thereof.

Examples of the pharmaceutically acceptable salt of the above compound (I) include an inorganic acid salt such as a hydrochloride, a sulfate or a hydrobromide, or an organic acid salt such as an acetate, a fumarate, an oxalate, a methanesulfonate or a malate.

A preferred embodiment of the compound (I) as an active ingredient of the present invention can be a compound of the following formula (I-A):

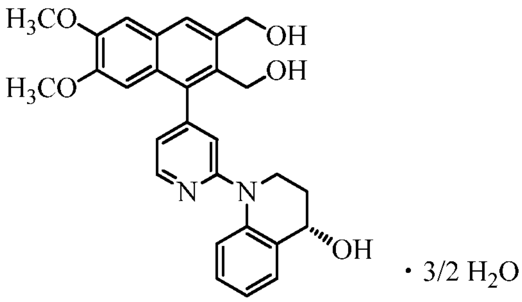

(I-A)

The pharmaceutical composition (antipruritic agent) of the present invention shows an excellent inhibiting effect on itch induced by various causes. Concretely, the pharmaceutical composition of the present invention is useful for prophylaxis or treatment of itch that is not associated with inflammatory skin reactions, such as pruritus associated with primary biliary cirrhosis, chronic renal failure/renal dialysis, abnormal blood pressure, thyroid gland malfunction, aging, cancer, anemia, a parasitic disease, a psycho-neurologic disease, a drug-induced disease and/or pregnancy, or pruritus induced by a pruritogen such as histamine, and further useful for prophylaxis or treatment of intractable itch associated with chronic prurigo etc. Besides, the pharmaceutical composition (antipruritic agent) of the present invention is useful for inhibiting itch being resistant to a conventional medicament such as a steroid, an anti-inflammatory agent (e.g., an antihistamine) or an immunosuppressant (e.g., tacrolimus).

The compound (I), a hydrate thereof or a pharmaceutically acceptable salt thereof as an active ingredient of the present invention can be obtained by a known method such as that described in EP0748805A1 or WO2007/040240.

In the pharmaceutical composition (antipruritic agent) of the present invention, in addition to the Compound (I), a hydrate thereof or a pharmaceutically acceptable salt thereof which is an active ingredient, an additive for a pharmaceutical preparation such as an absorption enhancer, a pH adjusting agent, a preservative, a flavoring agent, a dispersing agent, a humectant, a stabilizer, an antiseptic, a suspending agent, a surfactant and the like, may be formulated alone or in combination of two or more in admixture, if desired.

As the absorption enhancer, there may be mentioned, for example, a monohydric alcohol having 20 or less carbon atoms (ethyl alcohol, isopropyl alcohol, stearyl alcohol and the like), pyrrolidone derivatives (2-pyrrolidone, 1-methyl-2-pyrrolidone and the like), ureas (urea, thiourea and the like), cyclodextrins ($\alpha$-cyclodextrin and the like), menthol, 1-dodecylazacycloheptan-2-one, calcium thioglycolate, limonene and the like. An amount of the absorption enhancer may vary depending on the dosage form, ingredients of the base and the like, and in general, it is desirably 0.1% by

weight or more, preferably 0.3% by weight or more for the purpose of effectively producing an absorption-enhancing action, and desirably 10% by weight or less, preferably 5% by weight or less for the purpose of reducing side effect.

Specific examples of the pH adjusting agent may be mentioned, for example, an inorganic acid such as hydrochloric acid, sulfuric acid, phosphoric acid and the like, an organic acid such as acetic acid, succinic acid, fumaric acid, malic acid and the like, a metal salt of these acids and the like. An amount of the pH adjusting agent to be formulated may vary depending on the dosage form, ingredients of the base and the like, and in general, it is preferably so formulated that a pH of the preparation becomes 4 to 8.

Specific examples of the preservative or antiseptic may be mentioned, for example, p-hydroxybenzoic acid, methylparaben, chlorobutanol, benzyl alcohol, methyl p-hydroxybenzoate and the like.

Specific examples of the flavoring agent may be mentioned, for example, menthol, rose oil, eucalyptus oil, d-camphor and the like, and specific examples of the dispersing agent may be mentioned, for example, sodium metaphosphate, potassium polyphosphate, silicic acid anhydride and the like.

Specific examples of the humectant may be mentioned, for example, propylene glycol, glycerin, sorbitol, sodium lactate, sodium hyaluronate and the like, and specific examples of the stabilizer may be mentioned, for example, sodium hydrogen sulfite, tocopherol, ethylenediamine tetraacetic acid (EDTA), citric acid and the like.

Specific examples of the suspending agent may be mentioned, for example, tragacanth powder, Gum Arabic powder, bentonite, sodium carboxymethyl cellulose and the like, and specific examples of the surfactant may be mentioned, for example, polyoxyethylene hydrogenated castor oil, sorbitan fatty acid ester such as sorbitan sesquioleate and the like, polyoxyl stearate and the like.

An antipruritic agent of the present invention can be used as a topical agent for the purpose of directly administering it to a dermatitis area, and a dosage form thereof may be mentioned, for example, an ointment, a cream, a lotion, a liniment, a cataplasm, a plaster, a patch, a hard plaster, a gel, a liquid and the like.

When the above-mentioned dosage form is an ointment or a cream, an oleaginous base or an emulsion base can be used as a base.

As the oleaginous base, there may be mentioned, for example, a hydrocarbon (a hydrocarbon having 12 to 32 carbon atoms, liquid paraffin, white vaseline, squalene, squalane, plastibase and the like), a higher alcohol (an aliphatic monohydric alcohol having 12 to 30 carbon atoms such as lauryl alcohol, cetyl alcohol, stearyl alcohol, oleyl alcohol and the like), a higher fatty acid (a saturated or unsaturated fatty acid having 6 to 32 carbon atoms such as palmitic acid and stearic acid), a higher fatty acid ester (a fatty acid ester such as mirystyl palmitate and stearyl stearate; an ester of a fatty acid having 10 to 32 carbon atoms such as lanolin and carnauba wax, and an aliphatic monohydric alcohol having 14 to 32 carbon atoms; an ester of a saturated or unsaturated fatty acid having 10 to 22 carbon atoms and glycerin, such as glyceryl monolaurate, and its hydrogenated product, and the like), a glycol (ethylene glycol, propylene glycol, polyethylene glycol and the like), a vegetable oil, an animal oil and the like.

As the emulsion base, there may be mentioned, for example, an oil-in-water base, a water-in-oil base, a suspension base and the like. As the oil-in-water base, there may be mentioned, for example, a base prepared by emulsifying or dispersing a component such as the above-mentioned lanolin, propylene glycol, stearyl alcohol, vaseline, silicone oil, liquid paraffin, glyceryl monostearate, polyethylene glycol and the like, in an aqueous phase in the presence or in the absence of a surfactant, and the like. As the water-in-oil base, there may be mentioned, for example, a base prepared by adding water to a component such as vaseline, a higher aliphatic alcohol, liquid paraffin and the like, in the presence of a nonionic surfactant, and emulsifying or dispersing the mixture, and the like. Also, as the suspension base, there may be mentioned, for example, an aqueous base prepared by adding a suspending agent such as starch, glycerin, a high viscosity carboxymethyl cellulose, carboxyvinyl polymer and the like to water to make a gel, and the like.

The pharmaceutical composition (antipruritic agent) of the present invention can be prepared by a conventional method for preparation of a topical preparation. For example, an ointment or a cream can be prepared by mixing and kneading, emulsifying or suspending raw materials of a base depending on the respective dosage form to prepare the base, adding an active ingredient(s) and various kinds of additives, and mixing them in a mixer such as a screw mixer and the like.

The pharmaceutical composition (antipruritic agent) of the present invention can be used in any form of lotion such as a suspension, emulsion and solution. As a base for the suspension type lotion, there may be mentioned, for example, a mixture of a suspending agent including gums such as gum arabic, gum tragacanth and the like, celluloses such as methylcellulose, hydroxyethylcellulose and the like, clays such as bentonite and the like with water, and the like. As a base for the emulsion type lotion, there may be mentioned, for example, a base in which water and an oily substance including a fatty acid such as stearic acid, oleic acid and the like, a higher alcohol such as stearyl alcohol, cetyl alcohol and the like, are emulsified, and the like. As a base for the solution type lotion, there may be mentioned, for example, water and an alcohol such as ethanol, glycerin, propylene glycol and the like. The lotion can be prepared, for example, by adding various base components to purified water, mixing and stirring the same, then, adding an active ingredient(s) and an additive(s) to the mixture, and subjecting to filtration, if necessary.

As a base for the liniment, there may be mentioned, for example, vegetable oils such as olive oil and the like,

alcohols such as ethanol, isopropanol and the like, or a mixture of the above with water, and the like. The liniment can be prepared, for example, by dissolving an active ingredient in the base, and adding an additive(s) for a preparation to the mixture if desired and mixing the same.

As a base for a cataplasm, there may be mentioned, for example, water-soluble polymers such as polyacrylic acid, polyvinyl alcohol and polyvinyl pyrrolidone and the like. The cataplasm can be prepared, for example, by mixing an active ingredient, the base and an optionally desired additive(s) for a preparation, heating the same and then cooling.

As a base for the plaster, patch or hard plaster, there may be used, for example, a support such as non-woven fabric and the like, an elastomer such as natural rubber, isoprene rubber and the like, a filler such as zinc flower, titanium oxide and the like, a tackifier such as a terpene resin and the like, a peeling agent such as vinyl acetate and the like, a softening agent such as liquid paraffin and the like, an anti-aging agent such as dibutylhydroxytoluene (BHT) and the like, in an optional combination thereof. The plaster, patch, hard plaster and the like can be prepared by the conventional manner such as a solution method, a thermocompression method and the like.

As the solvent for the preparation of the liquid, there may be mentioned, for example, water, ethanol, isopropyl alcohol, benzyl alcohol, polyethylene glycol (PEG400 and the like), propylene glycol, propylene carbonate or a mixture thereof, and the like. Also, said liquid may be used by impregnating gauze, a wound dressing and the like with it.

An amount of the active ingredient to be formulated into the above-mentioned preparation may vary depending on a form of the preparation, and, for example, in the case of an ointment or a cream, it is preferably 0.0025 to 5% by weight, more preferably 1.25 to 5% by weight, further preferably 2.5 to 5% by weight, and in the case of a liquid, it is preferably 0.1 to 200 mg/mL, more preferably 0.1 to 50 mg/mL, further preferably 0.2 to 20 mg/mL.

A dose of the pharmaceutical composition (antipruritic agent) of the present invention may be determined depending on a kind, site or severity etc. of itch, and an appropriate amount of the above-mentioned preparation may be, for example, topically applied to the suffered area once to several times per day.

## EXAMPLES

### Experiment 1

[Antipruritic effect of test compound on pruritogen-induced scratching model]

Method:

For the purpose of measuring scratching behavior (counts of scratching), a cylindrical magnet (diameter: 1 mm, length: 3 mm) was implanted into instep of right hindlimb of each mouse (ICR female mouse, 6 weeks old; Charles River Laboratories, Japan Inc.) under anesthesia at least three days before testing, and hair on her right dorsal neck was removed by a shaver under anesthesia the day before testing. After topical administration of a solution of test compound in acetone (3% w/v solution of 1-[2-(4S)-4-hydroxy-1,2,3,4-tetrahydroquinolin-1-yl]-4-pyridyl]-2,3-bis(dihydroxymet hyl)-6,7-dimethoxynaphthalene sesquihydrate, as hereinafter referred to as Compound A, in acetone; 50 μL) or a vehicle (acetone; 50 μL) to the right dorsal neck of the above-treated mouse, these mice were placed into a testing apparatus (MicroAct, Neuroscience Inc.). After one hour of the administration of the above-mentioned solution, to the right dorsal neck of each mouse was intradermally administered a solution (50 μL) of a pruritogen in saline [histamine: 300 nmol/50 μL, U-46619 (chemical name: 9,11-dideoxy-9α,11α-methanoepoxyprosta-5Z,13E-dien-1-oic acid, Cayman Chemical Company): 10 nmol/50 μL, Compound 48/80 (Sigma): 3 μg/50 μL, serotonin (Sigma): 100 nmol/50 μL, or Substance P (Peptide Institute Inc.): 300 nmol/50 μL] or saline. Immediately after the administration, scratching behavior (counts of scratching) was measured for 30 minutes by MicroAct.

Results:

The results of the testing are shown in the following Tables 1 to 5.

Table 1

| Test group | Pruritogen | Test compound | Counts of Scratching (beats/30 min.) | | |
|---|---|---|---|---|---|
| | | | Mean | Standard error | Statistical analysis |
| Control group (n = 10) | Null (saline) | Null | 232 | 57 | --- |

(continued)

| Test group | Pruritogen | Test compound | Counts of Scratching (beats/30 min.) | | |
|---|---|---|---|---|---|
| | | | Mean | Standard error | Statistical analysis |
| Pruritogen-treated group (n =10) | histamine | Null | 712 | 120 | ** |
| Test compound-treated group (n = 11) | | Compound A | 365 | 41 | # |
| **: p<0.01 (vs. control group; Student's t-test)<br>#: p<0.05 (vs. pruritogen-treated group; Student's t-test) | | | | | |

Table 2

| Test group | Pruritogen | Test compound | Counts of Scratching (beats/30 min.) | | |
|---|---|---|---|---|---|
| | | | Mean | Standard error | Statistical analysis |
| Control group (n = 13) | Null (saline) | Null | 244 | 59 | --- |
| Pruritogen-treated group (n = 15) | U-46619 | Null | 1667 | 174 | ** |
| Test compound-treated group (n = 15) | | Compound A | 1069 | 163 | # |
| **: p<0.01 (vs. control group; Student's t-test)<br>#: p<0.05 (vs. pruritogen-treated group; Student's t-test) | | | | | |

Table 3

| Test group | Pruritogen | Test compound | Counts of Scratching (beats/30 min.) | | |
|---|---|---|---|---|---|
| | | | Mean | Standard error | Statistical analysis |
| Control group (n = 15) | Null (saline) | Null | 299 | 48 | --- |
| Pruritogen-treated group (n = 16) | Compound 48/80 | Null | 741 | 61 | ** |
| Test compound-treated group (n = 15) | | Compound A | 521 | 47 | ## |
| **: p<0.01 (vs. control group; Student's t-test)<br>##: p<0.01 (vs. pruritogen-treated group; Student's t-test) | | | | | |

Table 4

| Test group | Pruritogen | Test compound | Counts of Scratching (beats/30 min.) | | |
|---|---|---|---|---|---|
| | | | Mean | Standard error | Statistical analysis |
| Control group (n = 8) | Null (saline) | Null | 303 | 91 | --- |
| Pruritogen-treated group (n = 10) | serotonin | Null | 1699 | 273 | ** |
| Test compound-treated group (n = 10) | | Compound A | 841 | 66 | ## |
| **: p<0.01 (vs. control group; Student's t-test)<br>##: p<0.01 (vs. pruritogen-treated group; Student's t-test) | | | | | |

Table 5

| Test group | Pruritogen | Test compound | Counts of Scratching (beats/30 min.) | | |
|---|---|---|---|---|---|
| | | | Mean | Standard error | Statistical analysis |
| Control group (n = 13) | Null (saline) | Null | 310 | 66 | --- |
| Pruritogen-treated group (n = 14) | Substance P | Null | 1074 | 93 | ** |
| Test compound-treated group (n = 15) | | Compound A | 777 | 93 | # |
| **: p<0.01 (vs. control group; Student's t-test)<br>#: p<0.05 (vs. pruritogen-treated group; Student's t-test) | | | | | |

Discussion:

It is clear that Compound A as an active ingredient of the present invention significantly inhibited pruritogen-induced scratching behavior (counts of scratching) and thereby said compound has a remarkable antipruritic activity.

Experiment 2

[Antipruritic effect of test compound in histamine-induced scratching model]

Method:

After topical administration of a 3% w/v solution of a test compound or a reference compound as described in the following Table 6 in acetone (50 μL) or a vehicle (acetone; 50 μL) onto right dorsal neck of ICR female mice similarly pre-treated as described in Experiment 1, these animals were placed into a testing apparatus (MicroAct). After one hour of the administration of said compound solution or vehicle, a solution (50 μL) of histamine in saline (300 nmol/50 μL) or saline (50 μL) was intradermally administered to right dorsal neck of each mouse. Immediately after the administration, scratching behavior (counts of scratching) was measured for 30 minutes by MicroAct.

Results:

Inhibition rate on scratching behavior of each test compound was evaluated in terms of the following formula based on the counts of scratching in the test group in which each test compound solution was administered followed by administration of a histamine solution in saline (counts of scratching in compound-treated group), and the counts of scratching in the test group in which a vehicle (acetone) was administered followed by administration of a histamine solution in saline (counts of scratching in vehicle-treated group).

Ratio of inhibition rate on scratching behavior of each test compound to that of Compound A was calculated as antipruritic activity (%). The results are shown in the following Table 7. For reference, PDE4 inhibitory activity of each compound ($IC_{50}$) which was measured by a conventional manner is also shown in such Table.

$$\text{Inhibition rate on scratching behavior (\%)} = \frac{\text{Counts of scratching in vehicle-treated group} - \text{Counts of scratching in compound-treated group}}{\text{Counts of scratching in vehicle-treated group}} \times 100$$

Table 6

| | | |
|---|---|---|
| 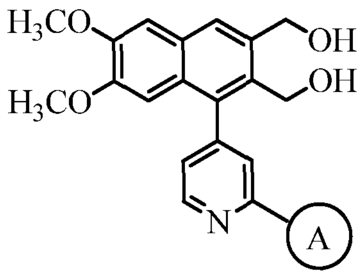 | | |
| | | Ring A |
| Test compound | Compound A | 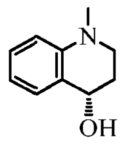 (sesquihydrate) |
| | Compound B | 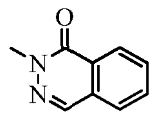 |
| | Compound C | 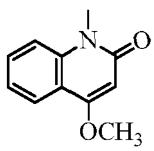 |
| | Compound D | 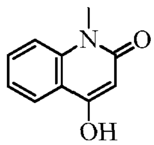 |
| Reference compound | Reference compound 1 | 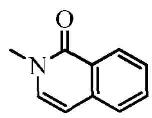 |
| | Reference compound 2 | 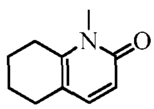 |
| | Reference compound 3 | 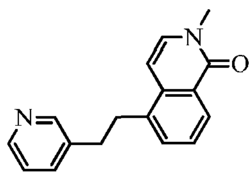 |

Table 7

| Test Compound | Antipruritic activity (%) | PDE4 inhibitory activity ($IC_{50}$; $\mu M$) |
|---|---|---|
| Compound A | 100 | 0.003 |
| Compound B | 154 | 0.001 |
| Compound C | 116 | 0.002 |

(continued)

| Test Compound | Antipruritic activity (%) | PDE4 inhibitory activity ($IC_{50}$; μM) |
|---|---|---|
| Compound D | 80 | 0.002 |
| Reference compound 1 | 14 | 0.001 |
| Reference compound 2 | -20 | 0.003 |
| Reference compound 3 | -36 | 0.0001 |

Discussion:

Whereas each of Compound A, Compound B, Compound C and Compound D as an active ingredient of the present invention showed a remarkable antipruritic effect in histamine-induced scratching model, such effect of Reference compound 1, 2 or 3 was very weak or was not observed.

Reference Experiment 3

[Evaluation of effectiveness and safety of test compound in an animal model]

(1) [Antipruritic activity of test compound in histamine-induced scratching model]

Method:

After topical administration of a solution (50 μL) of test compound (0.3 to 3% w/v solution of Compound A in acetone, or 0.03 to 0.3% w/v solution of cipamfylline in acetone), or a vehicle (acetone; 50 μL) onto right dorsal neck of ICR female mice similarly pre-treated as described in Experiment 1, these animals were placed into a testing apparatus (MicroAct). After one hour of the administration of said test compound solution or vehicle, a solution (50 μL) of histamine in saline (300 nmol/50 μL) or saline (50 μL; control group) was intradermally administered to right dorsal neck of each mouse. Immediately after the administration, scratching behavior (counts of scratching) was measured for 30 minutes by the testing apparatus (MicroAct). Results of the experiment are shown in the following Table 8.

(2) [Effect of test compound on spontaneous locomotor activity]

Method:

The same solution (50 μL) of test compound or a vehicle (50 μL) as described in the above-mentioned (1) was administered onto right dorsal neck of ICR female mice similarly pre-treated as described in Experiment 1. After one hour of the administration of the test compound solution or vehicle, these animals were placed into an apparatus for measuring spontaneous locomotor activity (SCANET, Melquest Ltd.), and their locomotor activity was measured for 30 minutes.

Results:

The results of measurement are shown in the following Table 9.

Table 8

| Test group | n | Counts of scratching (beats/30 min.) | | |
|---|---|---|---|---|
| | | Mean | Standard error | Statistical analysis |
| Control group | 12 | 265 | 57 | --- |
| Histamine-treated group | | 641 | 85 | ## |
| Compound A (0.3%)+Histamine-treated group | | 634 | 64 | N.S. |
| Compound A (1%)+Histamine-treated group | | 611 | 67 | |
| Compound A (3 %)+Histamine-treated group | | 314 | 36 | ** |

(continued)

| Test group | n | Counts of scratching (beats/30 min.) | | |
|---|---|---|---|---|
| | | Mean | Standard error | Statistical analysis |
| Control group | 8 | 255 | 51 | --- |
| Histamine-treated group | | 524 | 94 | # |
| Cipamfylline (0.03 %)+Histamine-treated group | | 579 | 100 | N.S. |
| Cipamfylline (0.1 %)+Histamine-treated group | | 369 | 156 | |
| Cipamfylline (0.3%)+Histamine-treated group | | 130 | 48 | * |
| ##: p<0.01, #: p<0.05 (vs Control group; Student's t-test)<br>**: p<0.01, *: p<0.05 (vs Histamine-treated group; Dunnett's test)<br>N.S.: Not significant (vs Histamine-treated group; Dunnett's test) | | | | |

Table 9

| Test group | n | Spontaneous locomotor activity (counts/30 min.) | | |
|---|---|---|---|---|
| | | Mean | Standard error | Statistical analysis |
| Control group (vehicle-treated group) | 12 | 6311 | 551 | --- |
| Compound A (0.3%)-treated group | | 5814 | 761 | N.S. |
| Compound A (1%)-treated group | | 5779 | 457 | |
| Compound A (3%)-treated group | | 6260 | 403 | |
| Control group (vehicle-treated group) | 8 | 7037 | 425 | --- |
| Cipamfylline (0.03%)-treated group | | 6760 | 627 | N.S. |
| Cipamfylline (0.1%)-treated group | | 5308 | 599 | |
| Cipamfylline (0.3%)-treated group | | 4924 | 605 | * |
| *: p<0.05 (vs Control group; Dunnett's test)<br>N.S.: Not significant (vs Control group; Dunnett's test) | | | | |

Discussion:

An active ingredient of the present invention (Compound A) did not show spontaneous locomotor activity lowering effect at a dose producing antipruritic effect (3% w/v). On the other hand, a PDE4 inhibitor cipamfylline showed a significant spontaneous locomotor activity lowering effect at a dose producing antipruritic effect (0.3% w/v). These results demonstrate that the antipruritic agent of the present invention comprising as an active ingredient Compound A is a medicament with low risks of central nervous system depressant action (adverse drug reactions).

Reference Experiment 4

[Antipruritic effect of test compound in chronic oxazolone-induced dermatitis model]

A magnet for measurement was implanted into an instep part of right hindlimb of BALB/c male mouse (fed for one week from five-week old; Charles River Laboratories, Japan Inc.) under anesthesia, and on the same day, sensitization was carried out by topically administering 0.5% w/v solution (10 μL) of oxazolone in acetone respectively onto the both sides of right ear auricle of the animal (sensitization day). On each point of the 7$^{th}$, 9$^{th}$, 11$^{th}$, 14$^{th}$ and 16$^{th}$ day reckoned from the day after the sensitization (1$^{st}$ day), each mouse was challenged by topical administration of a 10 μL solution of test compound [acetone (vehicle-treated group), 0.25% w/v solution of oxazolone in acetone (oxazolone-treated group), or a test compound solubilized in 0.25% w/v oxazolone solution in acetone (test compound-treated group)] onto the both sides of right ear auricle of the animal to produce inflammatory reactions. Immediately after the administration, scratching behavior (counts of scratching) was measured for two hours by MicroAct. Thickness of right

ear auricle of each mouse was measured by a thickness gauge before and 24 hours after the topical administration of each test compound solution. Meanwhile, the test compounds used in the experiment are shown in the following Table 10. The percentage described in the Table means "% w/v".

Table 10

| Test compound | Name of compound |
|---|---|
| Active ingredient of the present invention | Compound A (1%) |
| Reference compound 1 | Tacrolimus (0.1%) |
| Reference compound 2 | Dexamethasone (0.01%) |

Results:

Results of the measurement on right ear auricle thickness of mouse and the scratching behavior (counts of scratching) are shown in the following Tables 11 and 12 respectively.

Table 11

| Test compound (Test group) | Ear auricle thickness (Mean; mm) | | | | | | | | | | |
|---|---|---|---|---|---|---|---|---|---|---|---|
| | sensitization day | 7th day | 8th day | 9th day | 10th day | 11th day | 12th day | 14th day | 15th day | 16th day | 17th day |
| Vehicle | 0.27 | 0.27 | 0.28 | 0.27 | 0.28 | 0.27 | 0.27 | 0.27 | 0.27 | 0.27 | 0.27 |
| Oxazolone | 0.27 | 0.35 ## | 0.62 ## | 0.49 ## | 0.61 ## | 0.60 ## | 0.79 ## | 0.71 ## | 0.93 ## | 1.00 ## | 1.20 ## |
| Tacrolimus | 0.27 | 0.35 | 0.58 | 0.41 ** | 0.49 ** | 0.49 ** | 0.57 ** | 0.52 ** | 0.73 ** | 0.65 ** | 0.71 ** |
| Dexamethas one | 0.27 | 0.35 | 0.46 ** | 0.40 ** | 0.45 ** | 0.43 ** | 0.54 ** | 0.49 ** | 0.55 ** | 0.54 ** | 0.64 ** |
| Compound A | 0.27 | 0.35 | 0.44 ** | 0.39 ** | 0.46 ** | 0.44 ** | 0.48 ** | 0.44 ** | 0.54 ** | 0.47 ** | 0.50 ** |
| ##: p<0.01 (vs vehicle group; Student's t-test)<br>**: p<0.01 (vs oxazolone group; Dunnett's test) | | | | | | | | | | | |

Table 12

| Test group | Counts of scratching (beats/2 hr.) (Mean ± standard error) | | | | |
|---|---|---|---|---|---|
| | 7th day | 9th day | 11th day | 14th day | 16th day |
| Vehicle | 230 ± 38 | 127 ± 39 | 192 ± 32 | 270 ± 55 | 122 ± 17 |
| Oxazolone | 2161 ± 139 (##) | 2412 ± 402 (##) | 1540 ± 310 (##) | 2466 ± 338 (##) | 2520 ± 464 (##) |
| Tacrolimus | 1130 ± 135 (**) | 1655 ± 231 | 990 ± 148 | 1023 ± 148 (**) | 2164 ± 324 |
| Dexamethasone | 1663 ± 144 (*) | 1845 ± 442 | 1274 ± 296 | 1791 ± 354 | 2211 ± 430 |
| Compound A | 931 ± 135 (**) | 664 ± 99 (**) | 577 ± 77 (*) | 863 ± 122 (**) | 959 ± 166 (*) |
| ##: p<0.01 (vs vehicle group; Student's t-test)<br>**: p<0.01, *: p<0.05 (vs oxazolone group; Dunnett's test) | | | | | |

Discussion:

As shown in the above, whereas it was observed that each test compound inhibited inflammation on the 16th day, only Compound A was observed to produce an inhibitory effect on the counts of scratching (antipruritic effect)

among the test compounds. These results suggest that Compound A has an inhibitory activity of itch to which an immunosuppressant such as tacrolimus or a steroid such as dexamethasone is hardly effective (e.g., itch that is not associated with inflammatory skin reactions).

INDUSTRIAL APPLICABILITY

A pharmaceutical composition (antipruritic agent) of the present invention shows an excellent effect in which itch induced by various causes including itch not being induced by any inflammatory reaction can be inhibited thereby. Such antipruritic agent is useful for prophylaxis or treatment of itch associated with inflammatory skin disease including atopic dermatitis and psoriasis (itch that is associated with inflammatory reactions), and also useful for prophylaxis or treatment of itch that is not associated with inflammatory skin reactions, such as pruritus associated with primary biliary cirrhosis, chronic renal failure/renal dialysis, abnormal blood pressure, thyroid gland malfunction, aging, cancer, anemia, a parasitic disease, a psycho-neurologic disease, a drug-induced disease and/or pregnancy, or pruritus induced by a pruritogen such as histamine, and further useful for prophylaxis or treatment of itch associated with chronic prurigo. Besides, the pharmaceutical composition (antipruritic agent) of the present invention is useful for inhibiting itch being resistant to a conventional medicament such as a steroid, an anti-inflammatory agent (e.g., antihistamine) or an immunosuppressant (e.g., tacrolimus).

## Claims

1. A naphthalene compound for use in the prophylaxis or treatment of itch not induced by skin inflammatory reactions, having the following formula (I):

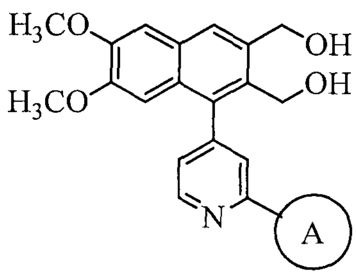

(I)

wherein the ring A is a heterocyclic group of the following formula:

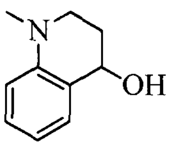

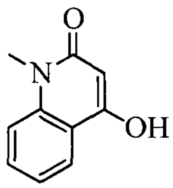

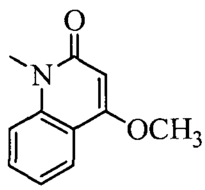

or

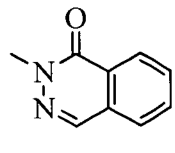

,

or a hydrate or a pharmaceutically acceptable salt thereof.

2. The naphthalene compound for use according to claim 1 in which the ring A is a heterocyclic group of the following formula:

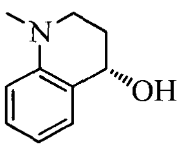

.

3. The naphthalene compound for use according to claim 1 in which the ring A is a heterocyclic group of the following formula:

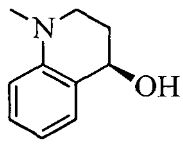

.

4. The naphthalene compound for use according to claim 1 having the following formula (I-A):

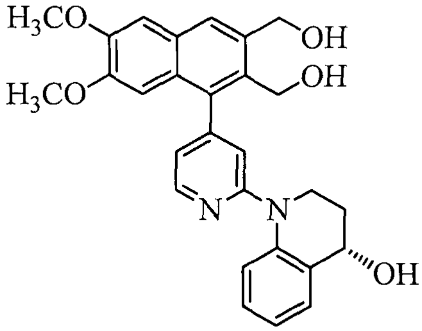

(I-A)

· 3/2 $H_2O$

.

5. A pharmaceutical composition for use in the treatment or prophylaxis of itch not induced by skin inflammatory reactions, comprising as an active ingredient the naphthalene compound according to any one of the claims 1-4, a hydrate thereof or a pharmaceutically acceptable salt thereof.

6. The naphthalene compound or pharmaceutical composition according to any one of claims 1 - 5 for use in the prophylaxis or treatment of pruritus.

7. The naphthalene compound or pharmaceutical composition according to any one of claims 1 to 5 for use in the prophylaxis or treatment of chronic prurigo.

8. The naphthalene compound or pharmaceutical composition for use according to claim 6 in which the pruritus is that associated with primary biliary cirrhosis, chronic renal failure/renal dialysis, abnormal blood pressure, thyroid gland malfunction, aging, cancer, anemia, a parasitic disease, a psycho-neurologic disease, a drug-induced disease and/or pregnancy, or pruritogen-induced pruritus.

9. The naphthalene compound or pharmaceutical composition for use according to claim 1 or 5 wherein the use is for inhibiting itch resistant to a steroid, an anti-inflammatory agent or an immunosuppressant.

10. The naphthalene compound or pharmaceutical composition for use according to any one of claims 1 to 5 which is for topical administration.

## Patentansprüche

1. Naphthalinverbindung zur Verwendung bei der Prophylaxe oder Behandlung von Juckreiz, der nicht durch entzündliche Hautreaktionen ausgelöst wird, mit der nachstehenden Formel (I):

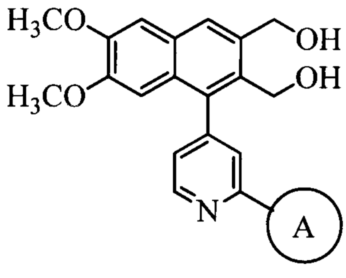

(I)

worin der Ring A eine heterocyclische Gruppe der nachstehenden Formel ist:

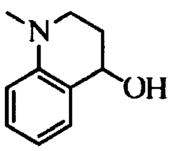 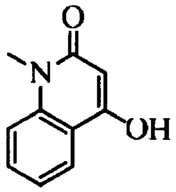 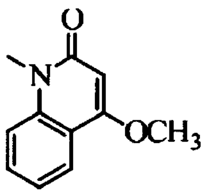 oder 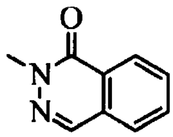 ,

oder ein Hydrat oder ein pharmazeutisch annehmbares Salz davon.

**2.** Naphthalinverbindung zur Verwendung gemäss Anspruch 1, in der der Ring A eine heterocyclische Gruppe der nachstehenden Formel ist:

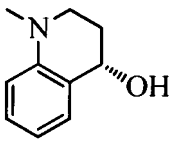

.

**3.** Naphthalinverbindung zur Verwendung gemäss Anspruch 1, in der der Ring A eine heterocyclische Gruppe der nachstehenden Formel ist:

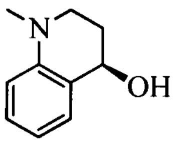

.

**4.** Naphthalinverbindung zur Verwendung gemäss Anspruch 1 mit der nachstehenden Formel (I-A):

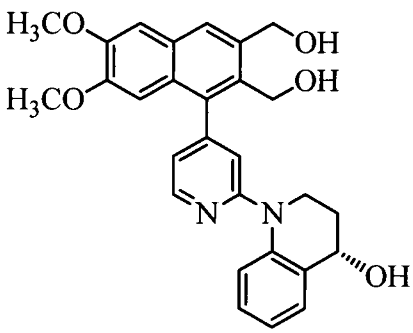

· 3/2 $H_2O$ (I-A)

.

**5.** Pharmazeutische Zusammensetzung zur Verwendung bei der Prophylaxe oder Behandlung von Juckreiz, der nicht durch entzündliche Hautreaktionen ausgelöst wird, die eine Naphthalinverbindung gemäss irgendeinem der Ansprüche 1 bis 4, ein Hydrat davon oder ein pharmazeutisch annehmbares Salz davon als Wirkstoff umfasst.

**6.** Naphthalinverbindung oder pharmazeutische Zusammensetzung gemäss irgendeinem der Ansprüche 1 bis 5 zur Verwendung bei der Prophylaxe oder Behandlung von Pruritus.

**7.** Naphthalinverbindung oder pharmazeutische Zusammensetzung gemäss irgendeinem der Ansprüche 1 bis 5 zur Verwendung bei der Prophylaxe oder Behandlung von chronischer Prurigo.

**8.** Naphthalinverbindung oder pharmazeutische Zusammensetzung zur Verwendung gemäss Anspruch 6, wobei der Pruritus mit primärer biliärer Zirrhose, chronischer Niereninsuffizienz/Nierendialyse, abnormalem Blutdruck, Schilddrüsenfehlfunktion, Alterung, Krebs, Anämie, einer parasitären Erkrankung, einer psychoneurologischen Erkrankung, einer Medikamenten-induzierten Erkrankung und/oder Schwangerschaft oder Pruritogen-induzierter Pruritus assoziiert ist.

9. Naphthalinverbindung oder pharmazeutische Zusammensetzung zur Verwendung gemäss Anspruch 1 oder 5 wobei die Verwendung zur Hemmung von Juckreiz dient, der gegen ein Steroid, ein entzündungshemmendes Mittel oder ein Immunsuppressivum resistent ist.

10. Naphthalinverbindung oder pharmazeutische Zusammensetzung zur Verwendung gemäss irgendeinem der Ansprüche 1 bis 5, die zur topischen Anwendung dient.

**Revendications**

1. Composé naphtalène pour l'utilisation dans la prophylaxie ou le traitement des démangeaisons non induites par des réactions inflammatoires de la peau, ayant la formula suivante (I) :

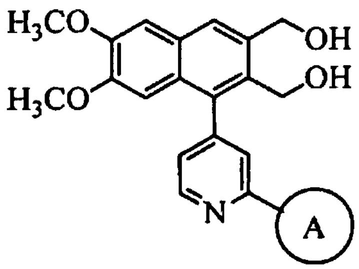

(I)

dans laquelle le cycle A est un groupe hétérocyclique de la formule suivante :

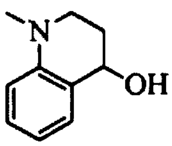

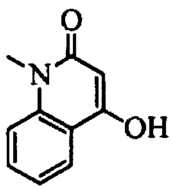

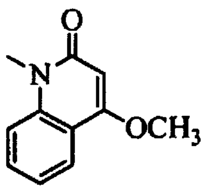

ou

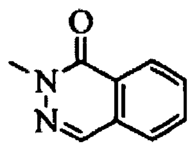

,

ou un hydrate ou un sel pharmaceutiquement acceptable de celui-ci.

2. Le composé naphtalène pour l'utilisation selon la revendication 1 dans lequel le cycle A est un groupe hétérocyclique de la formule suivante :

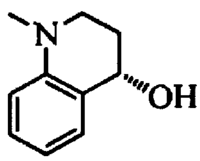

.

3. Le composé naphtalène pour l'utilisation selon la revendication 1 dans lequel le cycle A est un groupe hétérocyclique de la formule suivante :

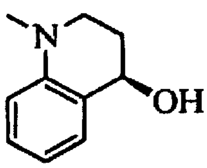

.

4. Le composé naphtalène pour l'utilisation selon la revendication 1 ayant la formule (I-A) suivante :

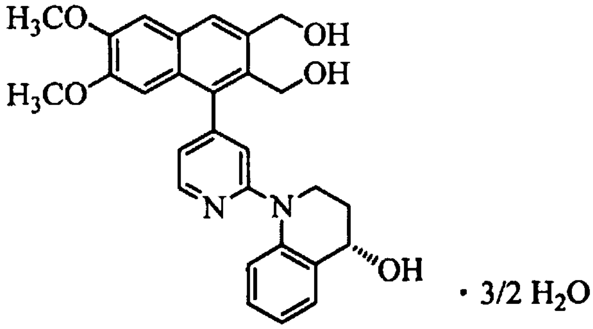

(I-A)

5. Composition pharmaceutique pour l'utilisation dans le traitement ou la prophylaxie des démangeaisons non induites par des réactions inflammatoires de la peau, comprenant en tant qu'ingrédient actif le composé naphtalène selon l'une quelconque des revendications 1-4, un hydrate de celui-ci ou un sel pharmaceutiquement acceptable de celui-ci.

6. Le composé naphtalène ou la composition pharmaceutique selon l'une quelconque des revendications 1-5 pour l'utilisation dans la prophylaxie ou le traitement du prurit.

7. Le composé naphtalène ou la composition pharmaceutique selon l'une quelconque des revendications 1-5 pour l'utilisation dans la prophylaxie ou le traitement du prurigo chronique.

8. Le composé naphtalène ou la composition pharmaceutique pour l'utilisation selon la revendication 6 dans laquelle le prurit est celui associé avec la cirrhose biliaire primaire, la dialyse rénale/l'insuffisance rénale chronique, la pression artérielle anormale, le dysfonctionnement de la glande thyroïde, le vieillissement, le cancer, l'anémie, un maladie parasitaire, une maladie psycho-neurologique, une maladie induite par un médicament et/ou la grossesse, ou le prurit induit par un pruritogène.

9. Le composé naphtalène ou la composition pharmaceutique pour l'utilisation selon la revendication 1 ou 5 dans laquelle l'utilisation est pour l'inhibition des démangeaisons résistantes à un stéroïde, un agent anti-inflammatoire ou un immunosuppresseur.

10. Le composé naphtalène ou la composition pharmaceutique pour l'utilisation selon l'une quelconque des revendications 1 à 5 qui est pour l'administration topique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0748805 A1
- WO 2007043426 A
- JP 2005047909 A
- WO 99020280 A
- WO 2007040240 A